# EUROPEAN PATENT APPLICATION

(11) **EP 4 810 596 A1**
(43) Date of publication of application: **23.09.2026**
(21) Application number: 26163128.7
(22) Date of filing: 09.03.2026
(51) Int. Cl.: C12M 1/107, C12M 1/34, C12M 1/36

(54) **BIO-METHANATION METHOD AND SYSTEM**

(30) Priority: 20.03.2025 DE 102025110900
(71) Applicant: ELECTROCHAEA GMBH, 82152 Planegg (DE)
(72) Inventor: Patel, Nitant, Planegg (DE); Cociancich, Matteo, Planegg (DE); Erben, Johannes, Planegg (DE); Quejigo, Jose Rodrigo, Planegg (DE)
(74) Representative: Fleuchaus & Gallo Partnerschaft mbB

(57) **Abstract**

The invention provides a system, method and controller for controlling a bio-methanation system. The method comprises: measuring dissolved gas partial pressure of a first gas in liquid contents of a bio-methanation reactor of the bio-methanation system with one or more first dissolved gas partial pressure sensors, wherein the first gas is H₂ or CH₄; measuring dissolved gas partial pressure of a second gas in the liquid contents of the bio-methanation reactor with one or more second dissolved gas partial pressure sensors, wherein the second gas is CO₂; determining with a controller in data communication with the one or more first dissolved gas partial pressure sensors and the one or more second dissolved gas partial pressure sensors whether a first ratio of or derived from measured partial pressures of the first and second gases is equal to a second ratio of partial pressures of the first and second gases comprising or derived from a preset value or values to within a preset tolerance; and responding to determining that the first ratio is not equal to the second ratio to within the preset tolerance by the controller controlling at least one of the first and second control valves and thereby adjusting at least one of the volumetric flow rates of H₂ and of CO₂ into the bio-methanation reactor so as to set the first ratio to be equal to the second ratio.

## Description

### Field of the Invention

The present invention relates to a bio-methanation method and system, and in particular to a method, system and controller for bio-methanation reactor stabilization.

### Background of the Invention

Bio-methanation reactors are controlled to operate within an optimal band of pressure and temperature conditions. One known technique for maintaining those conditions monitors CO₂ produced by the reactor, which should-under ideal operational condition-remain within a narrow range of values. According to this technique, a CO₂ partial pressure sensor is located above the liquid contents of the reactor and configured to monitor CO₂ partial pressure during reactor operation. This information is used by the reactor's controller to determine whether the feed gas ratio of the feed gases (H₂ and CO₂) should be modified and to what degree. The 'feed gas ratio' of H₂ and CO₂, as admitted to the reactor, is typically approximately four. That is, $\frac{{\dot{V}}_{{H}_{2}}}{{\dot{V}}_{{CO}_{2}}} \approx 4$, where *V̇*_{*H*₂} and *V̇*_{*CO*₂} are the volumetric flow rates (hereinafter referred to simply as 'flow rates') of H₂ and CO₂ respectively, such that the mole ratio is likewise ≈ 4. This ratio, $\frac{{\dot{V}}_{{H}_{2}}}{{\dot{V}}_{{CO}_{2}}}$, is adjusted by the reactor's controller in response to any significant deviation of the CO₂ partial pressure above the liquid contents of the reactor from optimal.

This technique has the advantage of relying on the actual performance of the reactor determined empirically by the CO₂ partial pressure sensor. However, it involves a delay between any modification in the flow of the feed gases and the response to that modification as determined by the CO₂ partial pressure sensor.

It is an object of the present invention to provide an improved method and system for bio-methanation reactor stabilization.

### Summary of the Invention

In a first aspect, the invention provides a bio-methanation system, comprising:
a bio-methanation reactor;
a first control valve configured to regulate a volumetric flow rate of H₂ into the bio-methanation reactor;
a second control valve configured to regulate a volumetric flow rate of CO₂ into the bio-methanation reactor;
a controller in data communication with the first control valve and the second control valve;
one or more first dissolved gas partial pressure sensors configured to measure dissolved gas partial pressure of a first gas in liquid contents of the bio-methanation reactor and to output first partial pressure sensor data indicative thereof, and in data communication with the controller, wherein the first gas is H₂ or CH₄; and
one or more second dissolved gas partial pressure sensors configured to measure dissolved gas partial pressure of a second gas in the liquid contents of the bio-methanation reactor and to output second partial pressure sensor data indicative thereof, and in data communication with the controller, wherein the second gas is CO₂;
wherein the controller comprises or is configured to access preset partial pressure values of the first gas and preset partial pressure values of the second gas, or of a preset value of a ratio of partial pressures of the first gas and the second gas, to receive the first partial pressure sensor data and the second partial pressure sensor data, to compare a first ratio of or derived from measured partial pressures of the first and second gases with a second ratio of partial pressures of the first and second gases comprising or derived from a preset value or values, and to respond to determining that the first ratio is not equal to the second ratio to within a preset tolerance by controlling at least one of the first and second control valves and thereby adjust at least one of the volumetric flow rates of H₂ and of CO₂ into the bio-methanation reactor so as to set the first ratio to be equal to the second ratio.

Thus, gas partial pressures in the liquid contents are monitored, so that the controller has more immediate and accurate data concerning the ratio of those partial pressures and can respond promptly.

The system may comprise:
a plurality of dissolved gas H₂ partial pressure sensors and a plurality of the dissolved gas CO₂ partial pressure sensors; or
a plurality of dissolved gas CH₄ partial pressure sensors and a plurality of the dissolved gas CO₂ partial pressure sensors; or
a plurality of dissolved gas H₂ partial pressure sensors, a plurality of dissolved gas CH₄ partial pressure sensors and a plurality of the dissolved gas CO₂ partial pressure sensors.

In an embodiment, the controller is configured to adjust at least one (and in some embodiments both) of the volumetric flow rate of H₂ and the volumetric flow rate of CO₂ into the bio-methanation reactor on the basis that the first ratio is proportional to or substantially equal to a ratio of the volumetric flow rates of H₂ and CO₂ into the bio-methanation reactor.

In an embodiment, the system comprises at least one pH sensor configured to measure the pH of the liquid contents of the bio-methanation reactor and to output pH sensor data indicative thereof, and in data communication with the controller.

In an embodiment, the system includes a pressure sensor configured to measure a headspace pressure in a headspace above the liquid contents of the bio-methanation reactor, and in data communication with the controller.

In an embodiment, the system a user interface in data communication with the controller and controllable by a user to input or modify the set points.

In an embodiment, the system comprises a set of sensors comprising (a) at least one of the first dissolved gas partial pressure sensors, and (b) at least one of the second dissolved gas partial pressure sensors, whereby the set of sensors measures the CO₂ partial pressure and the H₂ and/or CH₄ partial pressure at approximately the same depth in the liquid contents of the bio-methanation reactor. In an example, the set of sensors is one of a plurality of like sets of sensors, each of the plurality of sets of sensors being configured to measure the CO₂ partial pressure and the H₂ and/or CH₄ partial pressure at respective common depths in the liquid contents of the bio-methanation reactor and at a different depth from the other set or sets of sensors.

In an embodiment, the controller is further configured to respond to partial pressure sensor data received from one or more of the partial pressure sensors and indicating that one or more of the dissolved gas partial pressure of a first gas and the dissolved gas partial pressure of the second gas in the liquid contents is below a respective preset minimum partial pressure threshold, by controlling at least one of the first and second control valves and thereby increase at least one of the volumetric flow rates of H₂ and of CO₂ into the bio-methanation reactor. In an example, the partial pressure sensor data is received from a set of partial pressure sensors that is located or is locatable in a top of the liquid contents of the bio-methanation reactor or above any other set of partial pressure sensors.

In a second aspect, the invention provides a method of controlling a bio-methanation system, comprising:
measuring dissolved gas partial pressure of a first gas in liquid contents of a bio-methanation reactor of the bio-methanation system with one or more first dissolved gas partial pressure sensors, wherein the first gas is H₂ or CH₄;
measuring dissolved gas partial pressure of a second gas in the liquid contents of the bio-methanation reactor with one or more second dissolved gas partial pressure sensors, wherein the second gas is CO₂;
determining with a controller in data communication with the one or more first dissolved gas partial pressure sensors and the one or more second dissolved gas partial pressure sensors whether a first ratio of or derived from measured partial pressures of the first and second gases is equal to a second ratio of partial pressures of the first and second gases comprising or derived from a preset value or values to within a preset tolerance; and
responding to determining that the first ratio is not equal to the second ratio to within the preset tolerance by the controller controlling at least one of the first and second control valves and thereby adjusting at least one of the volumetric flow rates of H₂ and of CO₂ into the bio-methanation reactor so as to set the first ratio to be equal to the second ratio.

In an embodiment, the method comprises:
measuring dissolved gas H₂ partial pressure and dissolved gas CO₂ partial pressure; or
measuring dissolved gas CH₄ partial pressure and dissolved gas CO₂ partial pressure; or
measuring dissolved gas H₂ partial pressure, dissolved gas CH₄ partial pressure and dissolved gas CO₂ partial pressure.

In an embodiment, the method comprises the controller adjusting at least one of the volumetric flow rate of H₂ and the volumetric flow rate of CO₂ into the bio-methanation reactor on the basis that the first ratio is proportional to a ratio of the volumetric flow rates of H₂ and CO₂ into the bio-methanation reactor.

In an embodiment, the method comprises measuring the pH of the liquid contents of the bio-methanation reactor with at least one pH sensor configured to measure the pH of the liquid contents of the bio-methanation reactor; and/or
measuring a headspace pressure in a headspace above the liquid contents of the bio-methanation reactor with a pressure sensor.

In an embodiment, the method comprises measuring the CO₂ partial pressure and the H₂ and/or CH₄ partial pressure at approximately the same depth in the liquid contents of the bio-methanation reactor with a set of sensors comprising at least one of the first dissolved gas partial pressure sensors and at least one of the second dissolved gas partial pressure sensors. In an example, the set of sensors is one of a plurality of like sets of sensors, and the method comprises measuring the CO₂ partial pressure and the H₂ and/or CH₄ partial pressure at respective common depths in the liquid contents of the bio-methanation reactor and at a different depth from the other set or sets of sensors.

In an embodiment, the method comprises the controller responding to partial pressure sensor data received from one or more of the partial pressure sensors and indicating that one or more of the dissolved gas partial pressure of a first gas and the dissolved gas partial pressure of the second gas in the liquid contents is below a respective preset minimum partial pressure threshold, by controlling at least one of the first and second control valves and thereby increasing at least one of the volumetric flow rates of H₂ and of CO₂ into the bio-methanation reactor. In an example, the partial pressure sensor data is received from a set of partial pressure sensors that is located in a top of the liquid contents of the bio-methanation reactor or above any other set of partial pressure sensors.

In a third aspect, the invention provides an electronic or computer-implemented controller for controlling a bio-methanation system, the controller being configured to:
receive from one or more first dissolved gas partial pressure sensors in data communication with the controller one or more measurements of dissolved gas partial pressure of a first gas in liquid contents of a bio-methanation reactor of the bio-methanation system, wherein the first gas is H₂ or CH₄;
receive from one or more second dissolved gas partial pressure sensors in data communication with the controller one or more measurements of dissolved gas partial pressure of a second gas in liquid contents of the bio-methanation reactor, wherein the second gas is CO₂;
determine, such as with a partial pressure ratio comparator of the feed gas controller, whether a first ratio of or derived from measured partial pressures of the first and second gases is equal to a second ratio of partial pressures of the first and second gases comprising or derived from a preset value or values to within a preset tolerance; and
respond to determining that the first ratio is not equal to the second ratio to within the preset tolerance by controlling, such as with suitably adapted control signals generated by an ingas valve control signal generator of the controller (based on, for example, an ingas flow rate adjustment generated by an ingas flow rate adjustment determiner of the controller), at least one of the first and second control valves and thereby adjusting at least one of the volumetric flow rates of H₂ and of CO₂ into the bio-methanation reactor so as to set the first ratio to be equal to the second ratio.

Typically, the controller comprises or is configured to access preset partial pressure values of the first gas and preset partial pressure values of the second gas, or of a preset value of a ratio of partial pressures of the first gas and the second gas.

The measurements may comprise:
measurements of dissolved gas H₂ partial pressure and dissolved gas CO₂ partial pressure; or
measurements of dissolved gas CH₄ partial pressure and dissolved gas CO₂ partial pressure; or
measurements of dissolved gas H₂ partial pressure, dissolved gas CH₄ partial pressure and dissolved gas CO₂ partial pressure.

The controller may be configured to adjust at least one of the volumetric flow rate of H₂ and the volumetric flow rate of CO₂ into the bio-methanation reactor on the basis that the first ratio is proportional to a ratio of the volumetric flow rates of H₂ and CO₂ into the bio-methanation reactor.

The controller may be configured to receive:
measurements of the pH of the liquid contents of the bio-methanation reactor from at least one pH sensor configured to measure the pH of the liquid contents of the bio-methanation reactor; and/or
measurements of a headspace pressure in a headspace above the liquid contents of the bio-methanation reactor from a pressure sensor arranged to measure the headspace pressure.

The controller may be configured to receive measurements of the CO₂ partial pressure and the H₂ and/or CH₄ partial pressure at approximately the same depth in the liquid contents of the bio-methanation reactor from a set of sensors comprising at least one of the first dissolved gas partial pressure sensors and at least one of the second dissolved gas partial pressure sensors. In one example, the set of sensors is one of a plurality of like sets of sensors, and the controller is configured to receive measurements of the CO₂ partial pressure and the H₂ and/or CH₄ partial pressure at respective common depths in the liquid contents of the bio-methanation reactor and at a different depth from the other set or sets of sensors.

The controller may be configured to respond to partial pressure sensor data received from one or more of the partial pressure sensors and indicating that one or more of the dissolved gas partial pressure of a first gas and the dissolved gas partial pressure of the second gas in the liquid contents is below a respective preset minimum partial pressure threshold, by controlling at least one of the first and second control valves and thereby increasing at least one of the volumetric flow rates of H₂ and of CO₂ into the bio-methanation reactor. In one example, the controller receives the partial pressure sensor data from a set of partial pressure sensors that is located in a top of the liquid contents of the bio-methanation reactor or above any other set of partial pressure sensors.

The controller may be configured so as to combine any one or more of the features of the above embodiments.

It should be noted that any of the various individual features of each of the above aspects of the invention, and any of the various individual features of the embodiments described herein including in the claims, can be combined as suitable and desired.

### Brief Description of the Drawing

The invention is further described by way of the following detailed description in conjunction with the drawing. In the following detailed description, like reference numerals denote like elements except where indicated otherwise.

While the following detailed description describes certain embodiments of the invention in greater detail, it is noted that features described in context with only one of the embodiments are nevertheless intended to be available also in the context of or in combination with any other embodiment of the invention whether described or not in the detailed description, except where such a combination of features would lead to non-meaningful results. In no way is the following detailed description meant to limit the invention to the specific embodiments and combinations of features therein; rather, the invention is exclusively limited and defined by the attached claims.
Fig. 1 is a schematic view of a bio-methanation system according to a first embodiment of the present invention;
Fig. 2 is a schematic view of the feed gas controller of the bio-methanation system of Fig. 1 according to an embodiment of the present invention;
Fig. 3 is a flow diagram of a method of controlling the bio-methanation system of Fig. 1 according to an embodiment of the present invention;
Fig. 4A is a schematic view of a bio-methanation system according to a second embodiment of the present invention; and
Fig. 4B is a schematic view of a bio-methanation system according to a third embodiment of the present invention.

### Detailed Description

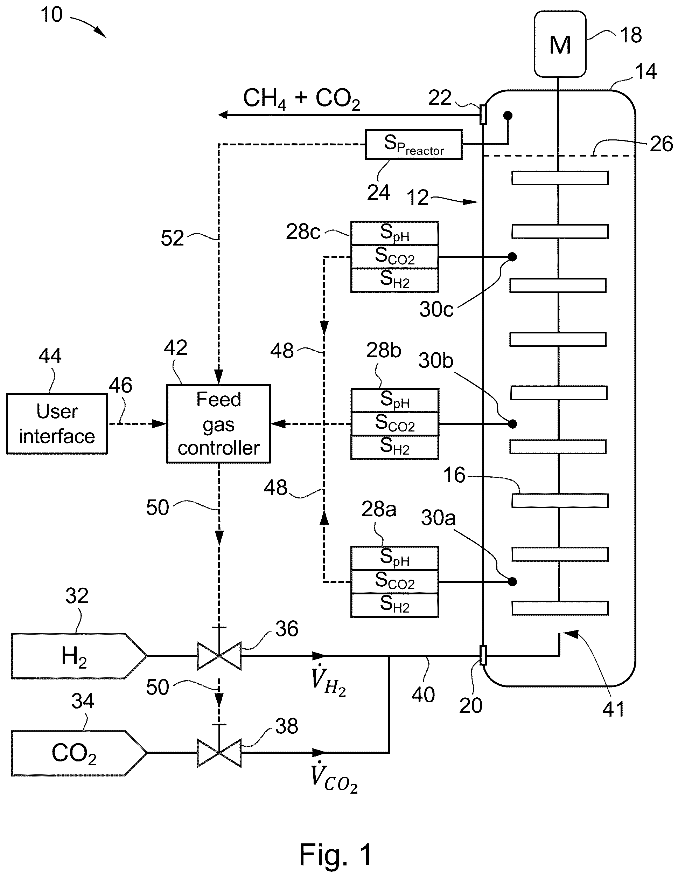

Fig. 1 is a schematic view of a bio-methanation system 10 according to a first embodiment of the present invention. Bio-methanation system 10 includes a bio-methanation reactor 12, which in turn includes a reactor vessel 14, agitator blades 16, a motor 18 for rotating the agitator blades 16, an inlet 20 for admitting feed gases (H₂ and CO₂) and an outlet 22 for extracting biogas (viz. CH₄, H₂, CO₂, etc). Outlet 22 typically includes or is associated with a remotely controllable valve (not shown) so that the rate of outflow of the biogas can be controlled.

System 10 includes a pressure sensor 24 arranged to measure, in use, the headspace pressure *P*_{reactor} in the 'headspace', that is, the volume above the surface 26 of the liquid contents of vessel 14. System 10 also includes one or more (in this example, three) sensor sets 28a, 28b, 28c, each including a dissolved gas CO₂ partial pressure sensor and a dissolved gas H₂ partial pressure sensor (and additionally in this embodiment a pH sensor). Sensor sets 28a, 28b, 28c are arranged to measure, in use, the pH, the dissolved CO₂ partial pressure and the dissolved H₂ partial pressure in the liquid contents of vessel 14 at different depths. Sensor sets 28a, 28b, 28c have respective detector heads 30a, 30b, 30c spaced, for example, equidistant from one another. Each of detector heads 30a, 30b, 30c may be located, for example, between (or at depths between) adjacent pairs of agitator blades 16.

System 10 includes a H₂ supply 32 and a CO₂ supply 34 that are coupled via respective flow control valves 36, 38 to a common gas conduit 40 that feeds H₂ and CO₂ from H₂ supply 32 and CO₂ supply 34 into vessel 14 via inlet 20 to a gas conduit outlet 41 located below the lowermost of agitator blades 16.

System 10 also includes a feed gas controller 42 and an input in the form of a user interface 44: feed gas controller 42 is configured to receive (either by wired or wireless communication link 46) set points from user interface 44 stipulating the desired values of the volume flow rates of the feed gases (H₂ and CO₂), and of some or all of the quantities monitored within the liquid, in particular the partial pressures of H₂ and CO₂ within the liquid (and/or the ratio of the partial pressures of H₂ and CO₂ within the liquid). Feed gas controller 42 is also configured to receive (either by wired or wireless communication link 48) the measured values of pH, the dissolved CO₂ partial pressure and the dissolved H₂ partial pressure of the liquid contents of vessel 14 from sensor sets 28a, 28b, 28c.

Feed gas controller 42 is configured to determine whether there is any difference (with a preset tolerance) between the set points and the measured (or 'process') values and to respond to a determined difference outside that preset tolerance by controlling control valves 36, 38 (either by wired or wireless communication link 50) so as to modify the ingas flow rate of H₂ and/or CO₂ until the respective partial pressure set point (e.g. the ratio of the partial pressures of H₂ and CO₂ within the reactor liquid) and process value again agree to within the applicable preset tolerance.

For example, if the measured or process values of dissolved CO₂ partial pressure and H₂ partial pressure are determined by feed gas controller 42 to be in a ratio that differs from the ratio of the set point values (or the ratio is determined to differ from a set point ratio) by more than the applicable preset tolerance, feed gas controller 42 responds by adjusting control valve 36 and/or control valve 38 so as to return that ratio to the desired value. The appropriate adjustment(s) is determined on the basis of the following analysis.

If the partial pressure of a gas (such as CO₂, H₂ or CH₄) in the liquid is represented as *p*_{gas} and the mole fraction of that gas as *n*_{gas}, then *p*_{gas} = *n*_{gas} × *P*_{reactor} since the reactor pressure is constant for all gases.

As a result, at gas conduit outlet 41 within the reactor liquid, the feed gas ratio $\frac{{\dot{V}}_{{H}_{2}}}{{\dot{V}}_{{CO}_{2}}} ∝ \frac{{p}_{{H}_{2}}}{{p}_{{CO}_{2}}}$. For example, if feed gas controller 42 determines that the measured value of *p*_{*H*₂} or the measured ratio $\frac{{p}_{{H}_{2}}}{{p}_{{CO}_{2}}}$ is lower or higher than its set point, feed gas controller 42 may respond by respectively increasing or decreasing the H₂ ingas flow, *V̇*_{*H*₂}*.*

Alternatively, if feed gas controller 42 determines that the measured ratio of *p*_{*H*₂} and *p*_{*CO*₂} is lower or higher than that ratio's set point, feed gas controller 42 responds by increasing or reducing the ratio of ingas flows *V̇*_{*H*₂} and *V̇*_{*CO*₂}, either by increasing the former, reducing the latter, or a combination of both.

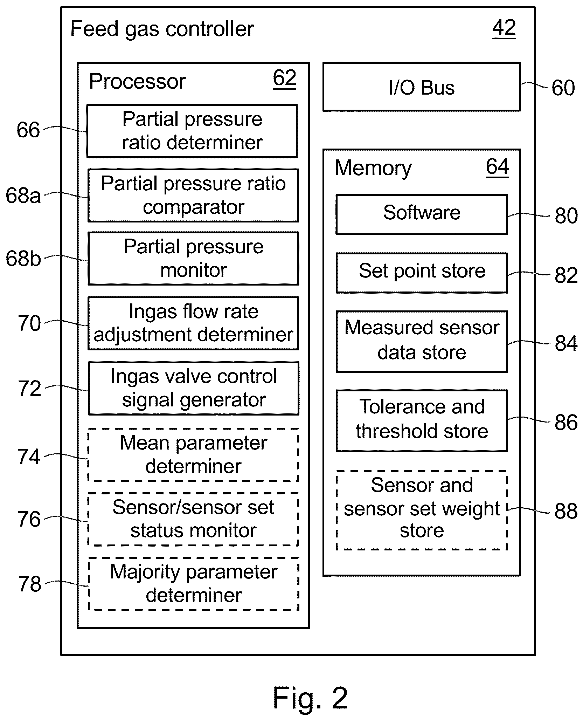

Fig. 2 is a simplified schematic view of feed gas controller 42 according to an embodiment of the present invention of the bio-methanation system of Fig. 1. Fig. 2 depicts only those components (whether implemented in software, firmware or otherwise) that facilitate the functions described herein. Thus, referring to Fig. 2, feed gas controller 42 includes an I/O bus 60, a processor 62 and memory 64 (which includes both volatile and non-volatile memory). Processor 64 includes a partial pressure ratio determiner 66, a partial pressure ratio comparator 68a, a partial pressure monitor 68b, an ingas flow rate adjustment determiner 70, an ingas valve control signal generator 72, a mean parameter determiner 74, a sensor/sensor set status monitor 76, and a majority parameter determiner 78. Memory 64 includes software 80 configured to, when executed, implement the lustrated components of processor 62, a set point store 82, a measured sensor data store 84, a tolerance and threshold store 86, and a sensor and sensor set weight store 88. The functions of these components-some of which are optional-are discussed below.

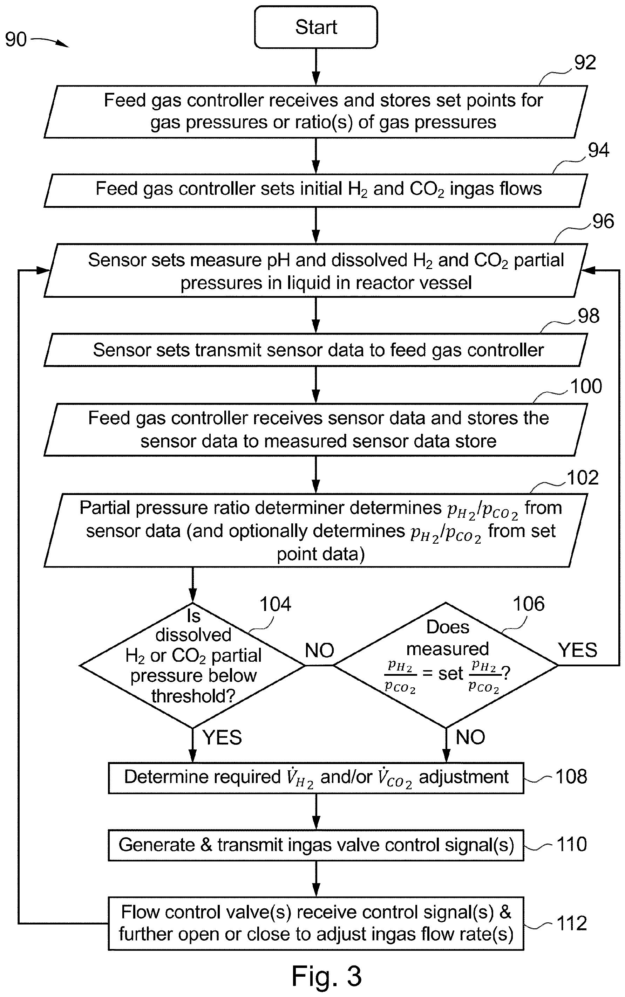

Fig. 3 is a flow diagram 90 of a method of controlling a bio-methanation system according to an embodiment of the present invention. At step 92, feed gas controller 42 receives set points for gas pressures or ratio(s) of gas pressures, such as from user interface 44 (e.g. input by a user) and stores the set points to set point store 82. The gas pressures are partial gas pressures of at least two dissolved gases. In this example those gases are H₂ and CO₂, (though H₂ and CH₄ are envisaged, as are H₂, CO₂ and CH₄, as discussed below).

At step 94, feed gas controller 42 sets valves 36, 38 so as to admit initial H₂ and CO₂ ingas flows to reactor vessel 14. These initial settings are selected to establish a desired ratio of flow rate of H₂ to flow rate of CO₂ of, in general, approximately 4, based on empirical data of the relationship between the settings of valves 36, 38 and the resulting flow rates or the resulting H₂ and CO₂ content of the liquid in reactor vessel 14.

At step 96, sensor sets 28a, 28b, 28c measure pH and dissolved H₂ and CO₂ partial pressures in the liquid in reactor vessel 14 and, at step 98, transmit the measured values as sensor data to feed gas controller 42.

At step 100, feed gas controller 42 receives the sensor data and stores that data in measured sensor data store 84. At step 102, partial pressure ratio determiner 66 of feed gas controller 42 responds by determining *p*_{*H*₂}/*p*_{*CO*₂} from the sensor data. If set point store 82 includes partial pressure set points rather than a *p*_{*H*₂}/*p*_{*CO*₂} ratio set point, pressure ratio determiner 66 also determines *p*_{*H*₂}/*p*_{*CO*₂} from the partial pressure set points.

At step 104, feed gas controller 42 checks whether it has received an alert from partial pressure monitor 68b indicating that the dissolved H₂ and/or CO₂ partial pressure is below a respective minimum partial pressure threshold (stored in tolerance and threshold store 86). This check is performed in case the ratio of partial pressures is acceptable but the absolute partial pressures are not. Partial pressure monitor 68b of feed gas controller 42 continually monitors the dissolved gas H₂ and CO₂ partial pressures and generates an alert if either or both are found to have fallen below a respective preset threshold. Partial pressure monitor 68b is configured to monitor the dissolved gas H₂ and CO₂ partial pressures received from uppermost sensor set 26c, because the H₂ and CO₂ partial pressures will taper downwards from the lower to the upper regions of the liquid contents of reactor vessel 14 as the H₂ and CO₂ are consumed in bio-methanation reactions. If either is exhausted in the upper region, the full capacity of the reactor volume is not being employed, so one or both ingas flows are desirably increased.

Thus, if at step 104 no such alert is detected (that is, neither of the dissolved H₂ and CO₂ partial pressures is below its respective preset minimum partial pressure threshold), processing continues at step 106. Otherwise, if either of the dissolved H₂ and CO₂ partial pressures is below its respective preset minimum partial pressure threshold, processing continues at step 108.

At step 106, partial pressure ratio comparator 68a of feed gas controller 42 determines whether the ratio *p*_{*H*₂}/*p*_{*CO*₂} determined from the sensor data equals (to within a preset tolerance retrieved from tolerance and threshold store 86) the ratio *p*_{*H*₂}/*p*_{*CO*₂} based on set point values retrieved from set point store 82 or determined in step 102. Changes in the ratio *p*_{*H*₂}/*p*_{*CO*₂} in the reactor can occur owing to the evolution of the conditions within reactor vessel 14 due to the chemical and biological processes in its contents. That evolution results in the continuous change in the pH and the partial pressures of H₂ and CO₂ is largely unpredictable ways. Changes in the ratio *p*_{*H*₂}/*p*_{*CO*₂} in the reactor can also be due to a malfunction of one or both flow control valves 36, 38.

If these measured and set point ratios are equal (to within the preset tolerance), reactor 12 is operating nominally so processing returns to step 96. Otherwise, processing continues at step 108.

At step 108, ingas flow rate adjustment determiner 70 determines the ingas flow rate adjustment required to restore reactor 12 to nominal operation by determining the ingas flow rate adjustment (of ingas flow rates *V̇*_{*H*₂} and/or *V̇*_{*CO*₂}) required to raise the dissolved H₂ and/or CO₂ partial pressure in the region of sensor set 28c to be above its respective preset minimum partial pressure thresholds and/or to maintain or restore the ratio $\frac{{p}_{{H}_{2}}}{{p}_{{CO}_{2}}}$ as measured to be equal to the ratio $\frac{{p}_{{H}_{2}}}{{p}_{{CO}_{2}}}$ based on the set point(s).

If the ratio $\frac{{p}_{{H}_{2}}}{{p}_{{CO}_{2}}}$ as measured is already equal to the ratio $\frac{{p}_{{H}_{2}}}{{p}_{{CO}_{2}}}$ based on the set point(s), ingas flow rate adjustment determiner 70 determines the appropriate increases in both ingas flow rates so that the dissolved H₂ and CO₂ partial pressures (as measured by sensor set 28c) will increase until both are above their respective minimum thresholds while maintaining the ratio $\frac{{p}_{{H}_{2}}}{{p}_{{CO}_{2}}}$ as measured. This is done by increasing the ingas flow rates by the same percentage (to maintain that ratio) and by absolute amounts known-whether empirically or based on theory-to effect the required increase in due course of the dissolved H₂ and/or CO₂ partial pressures.

If the ratio $\frac{{p}_{{H}_{2}}}{{p}_{{CO}_{2}}}$ as measured also requires adjustment, ingas flow rate adjustment determiner 70 advantageously determines adjustments that involve increasing one or both of the ingas flow rates, so as to achieve the desired set point ratio and to raise the dissolved H₂ and/or CO₂ partial pressures as required.

If only the ratio of partial pressures need be adjusted (that is, the dissolved H₂ and/or CO₂ partial pressures are acceptable), that ratio may be restored in several ways. For example, ingas flow rate adjustment determiner 70 may be configured to respond to the ratio *p*_{*H*₂}/*p*_{*CO*₂} based on the measured values being low by determining an adjustment configured to increase the ingas flow rate *V̇*_{*H*₂} and decrease the ingas flow rate *V̇*_{*CO*₂} by approximately the same amount (in either percentage or absolute terms); and to respond to the measured ratio *p*_{*H*₂}/*p*_{*CO*₂} (or ratio *p*_{*H*₂}/*p*_{*CO*₂} based on the measured values) being high by determining an adjustment configured to decrease the ingas flow rate *V̇*_{*H*₂} and increase the ingas flow rate *V̇*_{*CO*₂}*,* again by approximately the same amount (in either percentage or absolute terms). In another example, ingas flow rate adjustment determiner 70 is configured to respond to the measured *p*_{*H*₂}/*p*_{*CO*₂} (or ratio *p*_{*H*₂}/*p*_{*CO*₂} based on the measured values) being low by determining an adjustment configured to increase the ingas flow rate *V̇*_{*H*₂}; and to respond to the measured *p*_{*H*₂}/*p*_{*CO*₂} (or ratio *p*_{*H*₂}/*p*_{*CO*₂} based on the measured values) being high by determining an adjustment configured to increase the ingas flow rate *V̇*_{*CO*₂}*.*

Finite but unequal adjustments to the two ingas flows are also contemplated. In addition, preset minimum ingas flow rates are stored in tolerance and threshold store 86: ingas flow rate adjustment determiner 70 is configured to be contained by these minimum ingas flow rates when performing its determinations.

In step 108, therefore, ingas flow rate adjustment determiner 70 determines an adjustment of the ingas flow rate or rates as an amount or amounts that will restore the measured ratio $\frac{{p}_{{H}_{2}}}{{p}_{{CO}_{2}}}$ to that of the ratio $\frac{{p}_{{H}_{2}}}{{p}_{{CO}_{2}}}$ based on the set points, subject to the above constraints, based on the linear relationship between the feed gas ratio $\frac{{\dot{V}}_{{H}_{2}}}{{\dot{V}}_{{CO}_{2}}}$ and the partial pressure ratio $\frac{{p}_{{H}_{2}}}{{p}_{{CO}_{2}}}$. That adjustment is determined relative to or proportional to the initial or most recent settings of flow control valves 36, 38. For example, following the initial state of the liquid in reactor vessel 14 (at *t*₀), *p*_{*H*₂} and *p*_{*CO*₂} are generally measured at regular intervals, at *t*₀, *t*₁, *t*₂, *t*₃, etc. The required adjustment to the settings of flow control valves 36, 38 at *t*ₙ is determined relative to their initial settings (at *t*₀), or relative to the settings at *t*ₙ₋₁, on a proportional basis. If, for example, ingas flow rate adjustment determiner 70 determines at *t*ₙ that *V̇*_{*H*₂} should be doubled, ingas flow rate adjustment determiner 70 will determine an adjustment that opens flow control valves 36 to double its current setting, whether expressed as double the setting at *t*ₙ₋₁ or as an increment, decrement or multiple of the setting at *t*₀.

It will be appreciated that step 104 could in principle be implemented at any point in flow diagram 90. However, step 104 is advantageously implemented before step 106 in this embodiment so that ingas flow rate adjustment determiner 70 does not attempt to adjust the feed gas ratio $\frac{{\dot{V}}_{{H}_{2}}}{{\dot{V}}_{{CO}_{2}}}$ by reducing the ingas flow rate of a gas the dissolved partial pressure of which is below its minimum threshold.It should be noted that, advantageously, precise flow rates of *V̇*_{*H*₂} and *V̇*_{*CO*₂} are not required, as the control loop implemented in the above manner relies on feedback to maintain the desired partial pressure ratio and absolute partial pressures in the region of sensor set 28c. This avoids the difficulties of measuring these flow rates with any precision, or any reliance on assumptions about the relationship between the flow rates and the absolute valve settings of flow control valves 36, 38.

At step 110, ingas valve control signal generator 72 converts the ingas flow rate adjustment into one or more valve control signals, which it transmits to one or both flow control valves 36, 38, as appropriate.

At step 112, one or both flow control valves 36, 38 receive the valve control signal(s) and respond by further opening or further closing accordingly, and thereby adjusting one or both ingas flow rates.

Processing then returns to step 96.

In another embodiment, at step 108, ingas flow rate adjustment determiner 70 is configured to determine the required total adjustment but pass to ingas valve control signal generator 72 an adjustment that is either a preset fraction of that total adjustment or a preset minimum increment or decrement adjustment value. Processing then continues at step 110 and step 112, after which it returns, optionally after a delay sufficient for a new equilibrium (or near equilibrium) to be established within reactor vessel 14, to step 96. This embodiment has the advantage of reducing the likelihood of overshooting nominal operation or of too rapidly altering the H₂ or CO₂ content of the liquid in reactor vessel 14 in a manner that would flood or starve the bio-catalyst.

It should be emphasized that, in all embodiments, the set points may be for individual gases or for ratios of gases, or both.

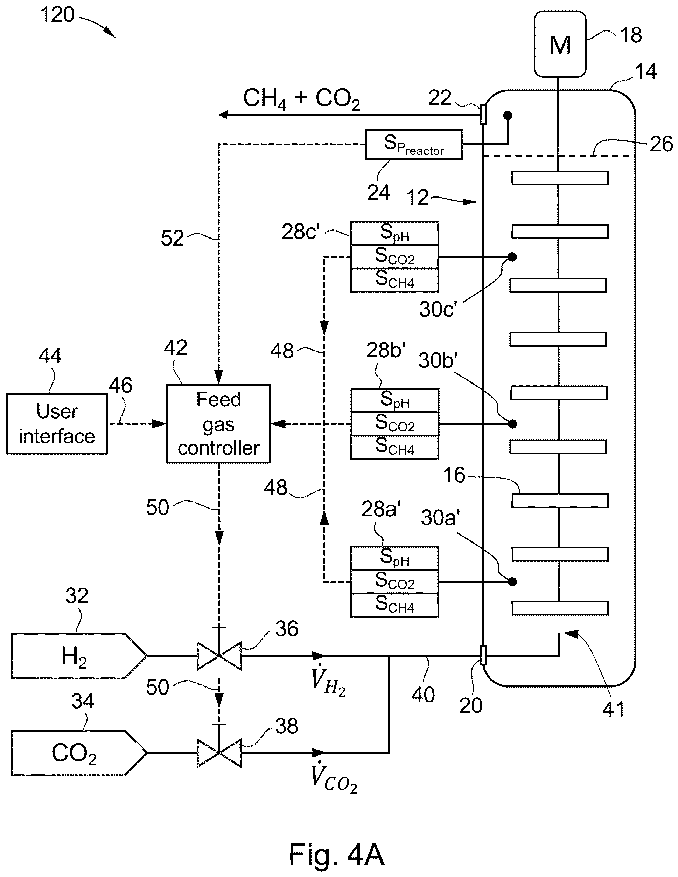

Fig. 4A is a schematic view of a bio-methanation system 120 according to a second embodiment of the present invention. Bio-methanation system 120 is identical in most respects with bio-methanation system 10 of Fig. 1, and like reference numerals have been used to identify like features.

However, instead of sensor sets 28a, 28b, 28c, bio-methanation system 120 includes sensor sets 28a', 28b', 28c' (with respective detector heads 30a', 30b', 30c'), each including a dissolved gas CO₂ partial pressure sensor, a dissolved gas CH₄ partial pressure sensor and a pH sensor. Feed gas controller 42 is configured to multiply CH₄ partial pressures in the liquid contents of reactor vessel 14 received from sensor sets 28a', 28b', 28c' by a factor of four and thereby derive corresponding values of H₂ partial pressure. This factor is based on the stoichiometric relationship between the species in the methanation reaction, 4H₂ + CO₂ → CH₄ + 2H₂O, but may be adjusted if a particular implementation is found to depart from that exact value.

Bio-methanation system 120 otherwise operates as described above.

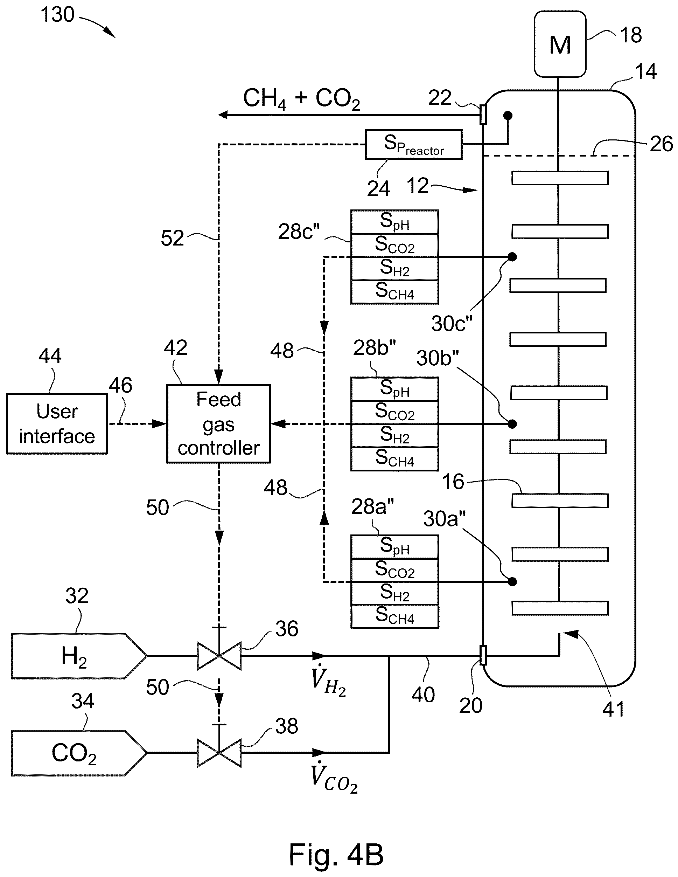

Fig. 4B is a schematic view of a bio-methanation system 120 according to a third embodiment of the present invention. Bio-methanation system 120 is also identical in most respects with bio-methanation system 10 of Fig. 1, and like reference numerals have been used to identify like features.

However, instead of sensor sets 28a, 28b, 28c, bio-methanation system 130 includes sensor sets 28a", 28b", 28c" (with respective detector heads 30a", 30b", 30c"), each including a dissolved gas CO₂ partial pressure sensor, a dissolved gas H₂ partial pressure sensor, a dissolved gas CH₄ partial pressure sensor and a pH sensor. Feed gas controller 42 is configured to multiply CH₄ partial pressures in the liquid contents of reactor vessel 14 received from sensor sets 28a", 28b", 28c" by a factor of four and thereby derive corresponding values of H₂ partial pressure (cf. bio-methanation system 120). Feed gas controller 42 is configured to use a derived H₂ partial pressure if the corresponding measured H₂ partial pressure is unavailable (such as should a dissolved gas H₂ partial pressure sensor fail or provide readings that appear to be aberrant), to check the measured H₂ partial pressure, and/or to detect any significant departure from the expected stoichiometric relationship-which may be indicative of contamination.

It should be understood that, in all of the above embodiments, the appropriate ingas flow rates *V̇*_{*H*₂} and *V̇*_{*CO*₂} are generallydependent on the specific characteristics of reactor 12 itself. In general, the flow of H₂ is desirably approximately four times that of CO₂ fed into reactor 12 and it is envisaged that the flow-rate adjustments discussed above entail small adjustments that do not modify that desired ratio excessively. In any case, that can be avoided by adjusting both ingas flow rates so that a ratio of approximately 4:1 (in this example) is maintained.

The outgas flow rate depends on the conversion to CO₂ to CH₄ in reactor 12, but is not required to be considered in the methodology described herein. However, the headspace pressure *P*_{reactor} is desirably maintained at a stable value, and certainly above a preset minimum during stable operation. Headspace pressure *P*_{reactor} is maintained above the preset minimum (stored in tolerance and threshold store 86), but it is not controlled using the ingas flow rates of the gases. Instead, pressure *P*_{reactor} is based on a set point set by the operator and controlled with a back pressure valve (not shown) that opens and closes based on a comparison of the set pressure and measured pressure-further opening when *P*_{reactor} is too high and partially closing when *P*_{reactor} is too low. Optionally, in those embodiments in which outlet 22 includes or is associated with a remotely controllable valve (as discussed above), the back pressure valve can be in the form of that valve.

In addition, it is desirable to maintain the pH and temperature of the contents of reactor vessel 14 in a range that is comfortable for biocatalyst, typically with a pH of 6.5 to 9 and a temperature of 65 °C ± 5 °C (or, more desirably, 63°C ± 2°C). Temperature is controlled directly, such as with a chiller or other head exchanger (not shown) that is controllable to reduce temperature when reactions within reactor 12 raise the temperature above the desired range. The pH is a function of dosing chemicals and feed gas flows so, although not actively controlled, is effectively controlled through the monitoring of CO₂ to H₂ partial pressures and the adjustment of ingas flow rates *V̇*_{*H*₂} and *V̇*_{*CO*₂} as described below.

Systems 10, 120 and 130 each includes three sensor sets, but other embodiments may include one or any desired plurality of sensor sets. However, employing a plurality of sensor sets has two principal advantages. Firstly, redundancy: the failure of any one set (or of communication with any one set) will not interrupt the operation of the system. Indeed, with plural sensor sets, two modes of operation are envisaged. For example, all sensor sets can be used simultaneously, with mean parameter determiner 74 of feed gas controller 42 configured to determine mean pH and pressure values (based on whichever one or more of the sensor sets are returning sensor data at any time) and feed gas controller 42 configured to control the system based on those mean values.

Alternatively, one or more (but not all) of the sensor sets can be employed with the one or more remaining sensor sets kept in reserve (whether powered on or off) until need upon failure of one or more of the initially operational sensor sets. For example, in one version of this configuration, each sensor set is assigned a rank or priority as primary, secondary, tertiary, etc, sensor set. The primary sensor set is initially employed; its status is continuously monitored with sensor/sensor set status monitor 76 and, if it is found to have failed, sensor/sensor set status monitor 76 controls feed gas controller 42 to employ data from the next available/functioning sensor set (e.g. the secondary sensor set). The status of that sensor set is then continuously monitored and, if it is found to have failed, sensor/sensor set status monitor 76 controls feed gas controller 42 to employ data from the next available/functioning sensor set (e.g. the tertiary sensor set), and so on. This simple approach maximizes the likelihood that the system will remain operational.

The second principal advantage is that employing plural sensor sets (28a, 28b, 28c; 28a', 28b', 28c', 28a"; 28b", 28c"), with respective detector heads located at different heights or depths in the reactor vessel 14, allows mean parameter determiner 74 to determine mean pH and pressure values with feed gas controller 42 configured to control the system based on those mean values-thereby reducing the effect of minor variations in pH or gas pressures within the reactor vessel.

This also allows feed gas controller 42 to be configured to ignore anomalous readings (e.g. a reading inconsistent with a plurality of other readings to a statistically significant degree), suggestive of possible sensor malfunction, by employing a 'voting' method-further increasing fault tolerance. In this approach, readings are collected from all three or more sensor sets for a given parameter (e.g. pH or the partial pressure of a specific gas). Majority parameter determiner 78 of feed gas controller 42 compares the readings and identifies the most common value within a preset tolerance (such as based on the known uncertainty in the readings or variability within the contents of the reactor vessel). The most common value by this test is selected by majority parameter determiner 78 as the basis for feed gas controller 42 to control ingas flows. Hence, if one sensor set gives a sporadic erroneous reading or fails entirely, the others should provide reliable values.

In those embodiments in which mean parameter determiner 74 determines mean pH and pressure values, it may also be desirable that mean values be calculated by mean parameter determiner 74 as weighted means, in which the weighting of each sensor set (and indeed sensor) is based on its reliability and performance history and is stored in sensor and sensor set weight store 88. The feed gas controller then computes a weighted average of the readings. The following summarizes the steps of this approach.
i) Assign initial weights (e.g., *w*₁, *w*₂, *w*₃) to each of the plurality of sensor sets. These weights can be based on historical accuracy, calibration data, or other reliability metrics.
ii) Collect readings from all of the plurality of sensor sets for a given parameter A (e.g. pH or the partial pressure of a specific gas).
iii) Determine the weighted mean *A̅* of the readings from the sensor sets, such as (in the example of three sensor sets): $\overline{A} = \frac{{w}_{1} ⋅ {A}_{1} + {w}_{2} ⋅ {A}_{2} + {w}_{3} ⋅ {A}_{3}}{{w}_{1} + {w}_{2} + {w}_{3}} .$

If a sensor set fails, its weighting as recorded in sensor and sensor set weight store 88 is set to zero. Further, the weightings can be either of an entire sensor set or of individual sensors.

This approach has the advantages of a) greater flexibility in handling sensor/sensor set reliability as less reliable sensor/sensor set can still be used but down-weighted, b) allowing dynamic adjustment of sensor/sensor set weighting based on real-time performance data, and c) smoothing anomalies by giving less weight to less reliable sensors/sensor sets.

Modifications within the scope of the invention may be readily effected by those skilled in the art. It is to be understood, therefore, that this invention is not limited to the particular embodiments described by way of example hereinabove.

In the claims that follow and in the preceding description of the invention, except where the context requires otherwise owing to express language or necessary implication, the word "comprise" or variations such as "comprises" or "comprising" is used in an inclusive sense, that is, to specify the presence of the stated features but not to preclude the presence or addition of further features in various embodiments of the invention.

Further, any reference herein to prior art is not intended to imply that such prior art forms a part of the common general knowledge in any country.

## Claims

1. A bio-methanation system, comprising:
a bio-methanation reactor;
a first control valve configured to regulate a volumetric flow rate of H₂ into the bio-methanation reactor;
a second control valve configured to regulate a volumetric flow rate of CO₂ into the bio-methanation reactor;
a controller in data communication with the first control valve and the second control valve;
one or more first dissolved gas partial pressure sensors configured to measure dissolved gas partial pressure of a first gas in liquid contents of the bio-methanation reactor and to output first partial pressure sensor data indicative thereof, and in data communication with the controller, wherein the first gas is H₂ or CH₄; and
one or more second dissolved gas partial pressure sensors configured to measure dissolved gas partial pressure of a second gas in the liquid contents of the bio-methanation reactor and to output second partial pressure sensor data indicative thereof, and in data communication with the controller, wherein the second gas is CO₂;
wherein the controller comprises or is configured to access preset partial pressure values of the first gas and preset partial pressure values of the second gas, or of a preset value of a ratio of partial pressures of the first gas and the second gas, to receive the first partial pressure sensor data and the second partial pressure sensor data, to compare a first ratio of or derived from measured partial pressures of the first and second gases with a second ratio of partial pressures of the first and second gases comprising or derived from a preset value or values, and to respond to determining that the first ratio is not equal to the second ratio to within a preset tolerance by controlling at least one of the first and second control valves and thereby adjust at least one of the volumetric flow rates of H₂ and of CO₂ into the bio-methanation reactor so as to set the first ratio to be equal to the second ratio.

2. A bio-methanation system as claimed in claim 1, comprising
a plurality of dissolved gas H₂ partial pressure sensors and a plurality of the dissolved gas CO₂ partial pressure sensors; or
a plurality of dissolved gas CH₄ partial pressure sensors and a plurality of the dissolved gas CO₂ partial pressure sensors; or
a plurality of dissolved gas H₂ partial pressure sensors, a plurality of dissolved gas CH₄ partial pressure sensors and a plurality of the dissolved gas CO₂ partial pressure sensors.

3. A bio-methanation system as claimed either claim 1 or 2, wherein the controller is configured to adjust at least one of the volumetric flow rate of H₂ and the volumetric flow rate of CO₂ into the bio-methanation reactor on the basis that the first ratio is proportional to or substantially equal to a ratio of the volumetric flow rates of H₂ and CO₂ into the bio-methanation reactor.

4. A bio-methanation system as claimed in any one of the preceding claims, comprising
at least one pH sensor configured to measure the pH of the liquid contents of the bio-methanation reactor and to output pH sensor data indicative thereof, and in data communication with the controller; and/or
a pressure sensor configured to measure a headspace pressure in a headspace within the bio-methanation reactor and above the liquid contents of the bio-methanation reactor, and in data communication with the controller.

5. A bio-methanation system as claimed in any one of the preceding claims, comprising a user interface in data communication with the controller and controllable by a user to input or modify the set points.

6. A bio-methanation system as claimed in any one of the preceding claims, comprising a set of sensors comprising (a) at least one of the first dissolved gas partial pressure sensors, and (b) at least one of the second dissolved gas partial pressure sensors, whereby the set of sensors measures the CO₂ partial pressure and the H₂ and/or CH₄ partial pressure at approximately the same depth in the liquid contents of the bio-methanation reactor.

7. A bio-methanation system as claimed in claim 6, wherein the set of sensors is one of a plurality of like sets of sensors, each of the plurality of sets of sensors being configured to measure the CO₂ partial pressure and the H₂ and/or CH₄ partial pressure at respective common depths in the liquid contents of the bio-methanation reactor and at a different depth from the other set or sets of sensors.

8. A bio-methanation system as claimed in any one of the preceding claims, wherein the controller is further configured to respond to partial pressure sensor data received from one or more of the partial pressure sensors, such as in the form of a set of partial pressure sensors that is located or is locatable in a top of the liquid contents of the bio-methanation reactor or above any other set of partial pressure sensors, and indicating that one or more of the dissolved gas partial pressure of a first gas and the dissolved gas partial pressure of the second gas in the liquid contents is below a respective preset minimum partial pressure threshold, by controlling at least one of the first and second control valves and thereby increase at least one of the volumetric flow rates of H₂ and of CO₂ into the bio-methanation reactor.

9. A method of controlling a bio-methanation system, comprising:
measuring dissolved gas partial pressure of a first gas in liquid contents of a bio-methanation reactor of the bio-methanation system with one or more first dissolved gas partial pressure sensors, wherein the first gas is H₂ or CH₄;
measuring dissolved gas partial pressure of a second gas in the liquid contents of the bio-methanation reactor with one or more second dissolved gas partial pressure sensors, wherein the second gas is CO₂;
determining with a controller in data communication with the one or more first dissolved gas partial pressure sensors and the one or more second dissolved gas partial pressure sensors whether a first ratio of or derived from measured partial pressures of the first and second gases is equal to a second ratio of partial pressures of the first and second gases comprising or derived from a preset value or values to within a preset tolerance; and
responding to determining that the first ratio is not equal to the second ratio to within the preset tolerance by the controller controlling at least one of the first and second control valves and thereby adjusting at least one of the volumetric flow rates of H₂ and of CO₂ into the bio-methanation reactor so as to set the first ratio to be equal to the second ratio.

10. A method as claimed in claim 9, comprising
measuring dissolved gas H₂ partial pressure and dissolved gas CO₂ partial pressure; or
measuring dissolved gas CH₄ partial pressure and dissolved gas CO₂ partial pressure; or
measuring dissolved gas H₂ partial pressure, dissolved gas CH₄ partial pressure and dissolved gas CO₂ partial pressure.

11. A method as claimed in either claim 9 or 10, comprising the controller adjusting at least one of the volumetric flow rate of H₂ and the volumetric flow rate of CO₂ into the bio-methanation reactor on the basis that the first ratio is proportional to a ratio of the volumetric flow rates of H₂ and CO₂ into the bio-methanation reactor.

12. A method as claimed in any one of claims 9 to 11, comprising
measuring the pH of the liquid contents of the bio-methanation reactor with at least one pH sensor configured to measure the pH of the liquid contents of the bio-methanation reactor; and/or
measuring a headspace pressure in a headspace above the liquid contents of the bio-methanation reactor with a pressure sensor arranged to measure the headspace pressure.

13. A method as claimed in any one of the claims 9 to 12, comprising measuring the CO₂ partial pressure and the H₂ and/or CH₄ partial pressure at approximately the same depth in the liquid contents of the bio-methanation reactor with a set of sensors comprising at least one of the first dissolved gas partial pressure sensors and at least one of the second dissolved gas partial pressure sensors, optionally with the set of sensors being one of a plurality of like sets of sensors with the method comprising measuring the CO₂ partial pressure and the H₂ and/or CH₄ partial pressure at respective common depths in the liquid contents of the bio-methanation reactor and at a different depth from the other set or sets of sensors.

14. A method as claimed in any one of the claims 10 to 15, comprising the controller responding to partial pressure sensor data received from one or more of the partial pressure sensors, such as in the form of a set of partial pressure sensors that is located in a top of the liquid contents of the bio-methanation reactor or above any other set of partial pressure sensors, and indicating that one or more of the dissolved gas partial pressure of a first gas and the dissolved gas partial pressure of the second gas in the liquid contents is below a respective preset minimum partial pressure threshold, by controlling at least one of the first and second control valves and thereby increasing at least one of the volumetric flow rates of H₂ and of CO₂ into the bio-methanation reactor.

15. An electronic or computer-implemented controller for controlling a bio-methanation system, the controller being configured to:
receive from one or more first dissolved gas partial pressure sensors in data communication with the controller one or more measurements of dissolved gas partial pressure of a first gas in liquid contents of a bio-methanation reactor of the bio-methanation system, wherein the first gas is H₂ or CH₄;
receive from one or more second dissolved gas partial pressure sensors in data communication with the controller one or more measurements of dissolved gas partial pressure of a second gas in liquid contents of the bio-methanation reactor, wherein the second gas is CO₂;
determine whether a first ratio of or derived from measured partial pressures of the first and second gases is equal to a second ratio of partial pressures of the first and second gases comprising or derived from a preset value or values to within a preset tolerance; and
respond to determining that the first ratio is not equal to the second ratio to within the preset tolerance by controlling at least one of the first and second control valves and thereby adjusting at least one of the volumetric flow rates of H₂ and of CO₂ into the bio-methanation reactor so as to set the first ratio to be equal to the second ratio.
